# EUROPEAN PATENT APPLICATION

(11) **EP 2 133 022 A1**
(43) Date of publication of application: **16.12.2009**
(21) Application number: 08157925.2
(22) Date of filing: 10.06.2008
(51) Int. Cl.: A61B 5/00

(54) **Method of detecting problems, device for imaging the interior of turbid media and computer program product**

(71) Applicant: Koninklijke Philips Electronics N.V., 5621 BA Eindhoven (NL); Philips Intellectual Property & Standards GmbH, 20099 Hamburg (DE)
(72) Inventor: The designation of the inventor has not yet been filed
(74) Representative: Damen, Daniel Martijn

(57) **Abstract**

A method of detecting technical problems in a device for imaging the interior of turbid media is provided. The method is for a device comprising: a receiving portion (2) for receiving a turbid medium (1) to be examined; at least one light source (6) optically connected to the receiving portion (2) for irradiating the interior of the receiving portion (2); and at least one detector (7) optically connected to the receiving portion (2) for detecting light emanating from the interior of the receiving portion (2); the at least one light source (6) and the at least one detector (7) being optically connected to the receiving portion (2) such that a plurality of different combinations of source position and detector position defining different light paths through the receiving portion (2) is provided. The method comprises: a measurement step with a turbid medium (1) accommodated in the receiving portion (2); the measurement step generating measurement data consisting of a plurality of measurement data values corresponding to a plurality of different light paths through the receiving portion (2); and a processing step for analyzing if technical problems have occurred during the measurement step. The processing step comprises: comparing the measurement data values to corresponding reference data values from a reference measurement with the receiving portion filled with an optically matching medium.

## Description

### FIELD OF INVENTION

The present invention relates to a method of detecting technical problems, to a device for imaging the interior of turbid media, and to a computer program product.

### BACKGROUND OF THE INVENTION

In the context of the present application, the term turbid medium is to be understood to mean a substance consisting of a material having a high light scattering coefficient, such as for example an intralipid solution or biological tissue. Further, light is to be understood to mean electromagnetic radiation of a wavelength in the range from 400 nm to 1400 nm. The term "optical properties" covers the reduced scattering coefficient µ'ₛ and the absorption coefficient µₐ. Furthermore, "matching optical properties" is to be understood as having a similar reduced scattering coefficient µ'ₛ and a similar absorption coefficient µₐ.

In recent years, several methods and devices for examining turbid media, e.g. female breast tissue, have been developed. In particular, new devices for detection and analysis of breast cancer have been developed and existing technologies have been improved. Breast cancer is one of the most occurring types of cancer: in 2002, for example, more than 1.1 million women were diagnosed and over 410.000 women died of breast cancer worldwide. Several types of devices for imaging the interior of a turbid medium by use of light have been developed. Examples for such devices are mammography devices and devices for examining other parts of human or animal bodies. A prominent example for a method for imaging the interior of a turbid medium is Diffuse Optical Tomography (DOT). In particular, such devices are intended for the in vivo localization of inhomogeneities in breast tissue of a part of a breast of a female human body. A malignant tumor is an example for such an inhomogeneity. The devices are intended to detect such inhomogeneities when they are still small, so that for example carcinoma can be detected at an early stage. A particular advantage of such devices is that the patient does not have to be exposed to the risks of examination by means of ionizing radiation, as e.g. X-rays.

WO 00/56206 A1 discloses a device for imaging the interior of a turbid medium by using a light source to irradiate the turbid medium and photodetectors for measuring a part of the light transported through the turbid medium. A control unit is provided for reconstructing an image of the interior of the turbid medium on the basis of the measured intensities. The disclosed device is particularly adapted for examining female breasts. In order to allow the examination of the turbid medium, the device is provided with a receptacle as a receiving portion enclosing a measuring volume and arranged to receive the turbid medium. Due to different sizes of the turbid media to be examined, the size of the receptacle for receiving the turbid medium does not perfectly match the size of the turbid medium, i.e. a space remains between the receptacle and the turbid medium.

The light used for examining the turbid medium has to be transmitted from the light source to the turbid medium and from the turbid medium to the photodetectors. A number of light paths coupling to the light source and a number of light paths coupling to photodetectors are distributed across the wall of the receptacle, i.e. ends of optical fibers acting as light guides are connected to the wall of the receptacle. The light source subsequently irradiates the turbid medium from different directions and the photodetectors measure a part of the light transmitted through the turbid medium. A plurality of such measurements are performed with the light directed to the turbid medium from different directions and, based on the results of the measurements, the control unit reconstructs the image of the examined turbid medium.

An optical matching medium for conducting optical energy generated by a light source at least from the light source to a turbid medium to be irradiated with at least a part of the optical energy generated by the light source is known from US patent 5,907,406. The known optical matching medium can be used for imaging an interior of a turbid medium, such as biological tissue, using diffuse optical tomography. In medical diagnostics the matching medium may be used, for instance, for imaging an interior of a female breast. In that case, at least a part of the turbid medium, in this case a female breast, may be accommodated in a receiving volume.

In US patent 5,907,406 the receiving volume is bounded by a cuplike wall portion. However, this is not always necessary. Inside the receiving volume, the part of the turbid medium under investigation is surrounded by the matching medium. The matching medium is chosen such that the optical parameters of the matching medium, such as the absorption and scattering coefficients, are substantially identical to the corresponding optical parameters of the turbid medium. In this way, image artifacts resulting from optical boundary effects that occur when light is coupled into and out of the turbid medium can be reduced. Furthermore, use of the matching medium prevents the occurrence of an optical short-circuit in the receiving volume around the turbid medium. An optical short-circuit occurs when light is detected that has propagated along a path inside the receiving volume but outside the turbid medium and, as a consequence, has not been sufficiently scattered and attenuated. In that case the intensity of the insufficiently scattered and attenuated detected light may dwarf the intensity of detected light that has been scattered and attenuated through passage through the turbid medium.

In the known devices for imaging the interior of turbid media comprising a receiving portion receiving the turbid medium and an optically matching fluid for filling a space between the turbid medium and the receiving portion, sometimes the problem occurs that the space between the turbid medium and the receiving portion, e.g. a cup-like receptacle, is not completely filled by the optically matching medium. For example, if not enough optically matching medium is present, a gas pocket, which in most cases will be an air pocket, is present in the space between the receiving portion and the turbid medium. Further, in particular if the optically matching medium is an optically matching fluid, gas bubbles may be present in the upper part of the space. Further, such an optically matching fluid may leak out during the measurement and inversely affect some of the source or detection positions. If such undesired inhomogeneities are present in the space between the turbid medium and the receiving portion during a measurement for imaging the interior of a turbid medium, serious measurement errors and artifacts in the reconstructed image follow. The presence of the undesired inhomogeneities only becomes clear after the measurement and evaluation of the results. Since the reconstruction of images from the measured data is rather slow, the images are usually analyzed long after data acquisition. I.e. in case of e.g. mammography, the patient has already left the hospital when a fluid problem can be identified in the reconstructed images. As a consequence, the repetition of the measurement is very inconvenient for the patient and disturbs the patient handling e.g. in a hospital considerably. Similar inconveniences can arise as a result of the presence of other technical problems.

### SUMMARY OF THE INVENTION

It is an object of the present invention to provide a method, a device and a computer program product enabling detection of technical problems in a fast and efficient way immediately after the measurement.

This object is solved by a method of detecting technical problems in a device for imaging the interior of turbid media according to claim 1. The method is for a device comprising: a receiving portion for receiving a turbid medium to be examined; at least one light source optically connected to the receiving portion for irradiating the interior of the receiving portion; and at least one detector optically connected to the receiving portion for detecting light emanating from the interior of the receiving portion; the at least one light source and the at least one detector being optically connected to the receiving portion such that a plurality of different combinations of source position and detector position defining different light paths through the receiving portion is provided. The method comprises a measurement step with a turbid medium accommodated in the receiving portion; the measurement step generating measurement data consisting of a plurality of measurement data values corresponding to a plurality of different light paths through the receiving portion; and a processing step for analyzing if technical problems have occurred during the measurement step. The processing step comprises: comparing the measurement data values to corresponding reference data values from a reference measurement with the receiving portion filled with an optically matching medium. The at least one detector and the at least one light source are arranged such that a plurality of different combinations of source position and detector position is provided. This can e.g. be achieved by providing a plurality of different detectors, by providing a plurality of different light sources, by providing a plurality of different light guides connecting to one or more light sources and/or to one or more detectors. For example, at least one light source can subsequently be connected to a plurality of different source positions; this can for instance be achieved by a fiber switch. However, it is also possible that a single source and a single detector are provided which are revolved around the receiving portion during the measurement. In this case for example, at any given moment during the measurement, there exists only one light path but, since the source and the detector are rotated during the measurement, over the whole measurement a plurality of different light paths through the receiving portion is provided. Corresponding reference data values is to be understood to mean reference data values acquired for identical combinations of source position and detection position.

Since, in the processing step, the measurement data values are compared to the corresponding reference data values, this comparison is performed on a point-by-point basis and can be performed very fast and efficiently immediately after the measurement without requiring complicated processing algorithms. Thus, it can be determined whether technical problems have occurred or not immediately after the actual measurement. As a consequence, in case of technical problems, these can be eliminated and the measurement can be repeated in a time-saving and cost-efficient way. Thus, information about technical problems which have occurred during the measurement, such as air bubbles in the receiving portion, is provided before a patient has moved away from the device for imaging the interior of turbid media. As a consequence, if necessary, the measurement can be repeated without delay.

If comparing the measurement data values to the corresponding reference data values is performed by calculating the ratios, particularly fast and efficient processing is achieved.

If the processing step is performed based on the measurement data before reconstruction of an image of the turbid medium, the decision can be made without waiting for the reconstruction of an image which takes a rather long time. Thus, the method is particularly fast and can be conveniently performed immediately after the measurement.

Preferably, in the processing step, only the measurement data values corresponding to directly neighboring source and detector positions are compared to the reference data values. In this case, due to the reduced set of data which is used, the processing can be performed very fast and immediately after the measurement. Further, by processing the measurement data values corresponding to directly neighboring source and detection positions, inhomogeneities near the cup wall (in particular air where it normally appears) can be detected based on the raw data of the measurement set of data acquired in the measurement step. This is based on the property that measurement data values acquired for directly neighboring light guides connecting to the source and the respective detector are almost only influenced by the optical properties of the medium directly surrounding the associated light guides. Thus, if the measurement data values for pairs of source position and detection position in which the source position and the detection position are directly adjacent is processed in a processing step, occurrence of voids in the measurement volume or other technical problems can be identified immediately after the measurement step. In particular with respect to air voids in the measurement volume, based on such processing, analysis of the optical properties near the boundary of the measurement volume, i.e. the inner wall surface of the receiving portion 2 where air accumulation is likely to occur, is possible.

Preferably, the processing step provides a result whether technical problems have occurred or not. This can for instance be achieved in an automated way using an image processing algorithm which presents the decision whether technical problems have occurred or not to an operator. Thus, the decision whether a technical problem has occurred or not can be provided in an automated way immediately after the measurement.

In the case that the processing step provides a graphical representation allowing an operator to decide if technical problems have occurred, the decision can be made immediately after the measurement by an operator by simply analyzing the graphical representation.

If a graphical representation of a comparison between the measurement results and the results from the reference measurement is provided, a reliable basis for the decision whether technical problems have occurred or not is provided. The comparison can for instance be performed by dividing the respective results and graphically representing the obtained ratio.

Preferably, the graphical representation is provided such that it indicates at which position in the receiving portion a technical problem has occurred. In this case, based on the information, the operator can quickly identify the cause of the technical problem and possibly remove this cause such that the measurement can be repeated in a convenient and cost-efficient way.

The object is also solved by a device for imaging the interior of turbid media according to claim 9. The device comprises: a receiving portion for receiving a turbid medium to be examined; at least one light source optically connected to the receiving portion for irradiating the interior of the receiving portion; and at least one detector optically connected to the receiving portion for detecting light emanating from the interior of the receiving portion. The at least one light source and the at least one detector are optically connected to the receiving portion such that a plurality of different combinations of source position and detector position defining different light paths through the receiving portion is provided. The device further comprises a control unit which is adapted to control the device such that: a measurement with a turbid medium accommodated in the receiving portion is performed, the measurement generating measurement data consisting of a plurality of measurement data values corresponding to a plurality of different light paths through the receiving portion; and a processing operation for analyzing if technical problems have occurred during the measurement step is performed, the processing operation comprising: comparing the measurement data values to corresponding reference data values from a reference measurement with the receiving portion filled with an optically matching medium. The device achieves similar advantages as the method. Since, in the processing step, measurement data values are compared to the corresponding reference data values, point-by-point processing is possible. Thus, the processing can be performed very fast and immediately after the measurement. Information about technical problems which have occurred during the measurement, such as air bubbles in the receiving portion, is provided before a patient has moved away from the device for imaging the interior of turbid media. As a consequence, if necessary, the measurement can be repeated without delay.

Preferably, the control unit is adapted such that, in the processing operation, only measurement data values corresponding to directly neighboring source and detector positions are compared. In this case, the set of data which has to be processed is further reduced. Further, the boundary part of the receiving portion (in which technical problems are most likely to occur) is reliably analyzed. Still further, a particularly convenient representation of the results of this processing can be provided to an operator.

Preferably, the at least one light source is optically connected to the receiving portion such that a plurality of different source positions is distributed around the receiving portion. Preferably, the at least one detector is optically connected to the receiving portion such that a plurality of different detection positions is distributed around the receiving portion. In these cases, a plurality of different source positions and/or detection positions is distributed around the measurement volume and local information about technical problems can be provided.

If source positions and detection positions are alternately distributed around the receiving portion, a large number of directly neighboring source and detection positions is provided and local information about technical problems can be provided with high spatial resolution.

Preferably, the device is a medical image acquisition device.

The object is also solved by a computer program product for a device for imaging the interior of turbid media. The computer program product is for such a device which comprises: a receiving portion for receiving a turbid medium to be examined; at least one light source optically connected to the receiving portion for irradiating the interior of the receiving portion; at least one detector optically connected to the receiving portion for detecting light emanating from the interior of the receiving portion; and a control unit controlling the operation of the device. The computer program product is adapted such that, when loaded in the control unit, the device is controlled such that a measurement step with a turbid medium accommodated in the receiving portion is performed, the measurement step generating measurement data consisting of a plurality of measurement data values corresponding to a plurality of different light paths through the receiving portion; and a processing step for analyzing if technical problems have occurred during the measurement step is performed, the processing step comprising: comparing the measurement data values to corresponding reference data values from a reference measurement with the receiving portion filled with an optically matching medium. The computer program product achieves the advantages as described with respect to the method. The computer program product can be provided stored on a data carrier such as CD-ROM, USB stick and the like; it can be provided to be downloadable from the internet or an intranet. Other forms of distribution are also possible.

### BRIEF DESCRIPTION OF THE DRAWINGS

Further features and advantages of the present invention will arise from the detailed description of embodiments with reference to the enclosed drawings.
Fig. 1 schematically shows a receiving portion of a device for imaging the interior of turbid media with a turbid medium and an optically matching medium located therein;
Fig. 2 schematically shows the optical connection of the receiving portion and the light source and detector, respectively.
Fig. 3 schematically shows the receiving portion of Fig. 1 for the case that undesired inhomogeneities are present between the turbid medium and the receiving portion.
Fig. 4a to 4c show exemplary contact maps.
Fig. 5a to 5d show exemplary contact maps visualizing increasing air voids inside a measurement volume.

### DETAILED DESCRIPTION OF EMBODIMENTS

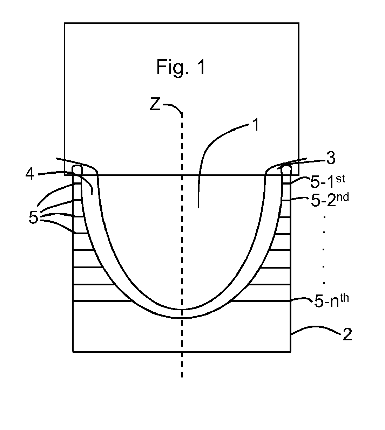

An embodiment of the present invention will now be described with reference to Figs. 1 to 3. In the embodiment, the device for imaging the interior of a turbid medium is formed by a device for diffuse optical tomography (DOT), in particular by a mammography device. Since the overall construction of such a device is known to a skilled person, no detailed description of the device will be given.

In the device of the embodiment, the turbid medium 1 to be examined is a female human breast. The device is provided with a cradle on which a patient is placed. In the cradle, a receiving portion 2 enclosing a measuring volume and arranged to receive the turbid medium 1 is provided, as schematically indicated in Fig. 1. The receiving portion 2 has a cup-like shape with rotational symmetry with respect to a vertical axis Z and is provided with an opening 3. As can be seen in Fig. 1, the turbid medium 1 to be examined, i.e. the breast, is placed in the receiving portion 2 such that it freely hangs in the receiving portion 2 from the side of the opening 3. The inner surface of the receiving portion 2 facing the turbid medium 3 is provided with a plurality of ends of light guides 5 formed by optically guiding fibers connecting to a light source 6 and to a plurality of detectors 7. These ends of the light guides 5 are distributed on the inner surface of the receptacle 2 such that the receptacle 2 provided with the light guides 5 still comprises substantially rotational symmetry. The ends of the light guides 5 on the side of the receiving portion 2 are arranged on a plurality of rings 5-1^{st}, 5-2^{nd},... , 5-n^{th} which are positioned in planes perpendicular to the axis Z, as schematically indicated in Fig. 1. On each ring, ends of a plurality of light guides 5 are distributed about the circumference of the receiving portion 2.

For example, in the device according to the embodiment 512 light guides 5 are provided the ends of which are distributed on the receiving portion 2. The light guides 5 may be formed by optically guiding fibers. In the embodiment, half of these light guides 5 are connected to an array of detectors 7. The other half of the light guides 5 is connected to a switch 9 capable to direct light from the light source 6 in either one of the 256 light guides 5. It should be noted that the number of light guides 5 is not limited to the number described above. Further, more or less than half of the light guides 5 may be connected to the detectors 7. In the present embodiment, the light source 6 is formed by a laser. However, more than one light source may be provided the light of which can be directed in selected light guides 5 by the switch 9.

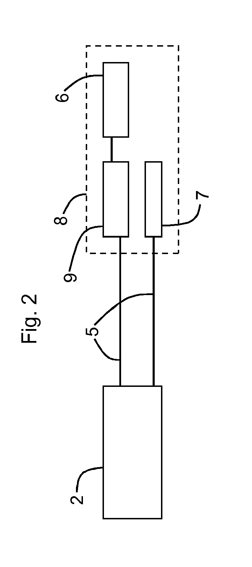

The device is structured such that light from the light source 6 can be subsequently directed to the turbid medium 1 from different directions and light emanating from the turbid medium 1 can be detected by a plurality of detectors 7 the corresponding light guides 5 of which are distributed on the inner surface of the receptacle 2. The device comprises a control unit 8 which reconstructs an image of the interior of the turbid medium 1 based on the signals from the detectors 7. For reconstruction, the signals sampled during a scan in which the light is directed to the turbid medium 1 from different directions are used. For reasons of simplicity, these elements of the device for imaging the interior of a turbid medium are only schematically indicated in Fig. 2. In Fig. 2, the control unit 8 comprises the light source 6 and the plurality of detectors 7. In the embodiment, for example the light from the light source 6 is subsequently directed into different light guides 5 and the light which emanates from the receiving portion 2 in response is detected by the plurality of detectors 7 in each case, e.g. 256 detectors.

The size of the receiving portion 2 is such that a space remains between the inner surface of the receiving portion 2 and the turbid medium 1. The receiving portion 2 is structured to receive an optically matching medium 4 for filling a space between an inner surface of the receiving portion 2 and the turbid medium 1. For examination, this space is filled with an optically matching medium 4 which serves to provide optical coupling between the turbid medium 1 to be imaged and the inner surface of the receiving portion 2. The optically matching medium 4 further serves to prevent optical short-cutting between the light guides 5 coming from the light source 6 and the light guides 5 coupling to the detectors 7. Furthermore, the optically matching medium 4 serves to counteract boundary effects in the reconstructed image which are caused by the difference in optical contrast between the interior of the turbid medium 1 and the remaining space in the receiving portion 2. For this purpose, the optically matching medium 4 is provided with optical properties which substantially match the optical properties of the turbid medium 1 to be examined.

In operation, i.e. for imaging the interior of a turbid medium, first a reference measurement with the receiving portion 2 completely filled with the optically matching medium 4 is performed. Thereafter, the turbid medium to be examined, e.g. the female human breast, is immersed in the optically matching medium 4 in the receiving portion 2 and the actual measurement is performed. Both, the reference measurement and the actual measurement consist of a large number of detector signals each forming a measurement set of data. For example, in the embodiment 256×256 detector signals form the measurement set of data, since 256 detectors and corresponding light guides 5 are provided and the light can be directed to the interior of the receiving portion 2 via 256 different light guides 5. In the case that a plurality of different light sources is provided, for example lasers emitting light of different wavelengths, this number of detector signals may be measured for each of the plurality of light sources such that the measurement set of data becomes even larger. The measured detector signals forming the measurement set of data can then be converted into a three-dimensional image of the interior of the turbid medium using a process called image reconstruction. Although the number of measured signals forming the measurement set of data is not restricted to the values described above it becomes clear that a measurement set of data consists of a large number of data. The process of reconstructing an image of the interior of the turbid medium requires a considerable time and e.g. is possibly performed after the patient has left the hospital in case of mammography.

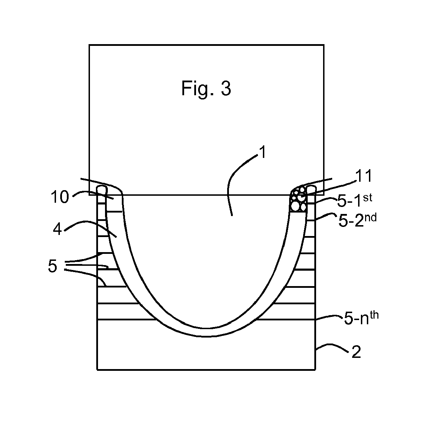

Since the turbid medium 1 to be examined has to be immersed in the optically matching medium 4 in the receiving portion 2 before the actual measurement is performed, a problem occurs that the space between the receiving portion 2 and the turbid medium 1 may not be completely filled with the optically matching medium 1, as schematically indicated in Fig. 3. For example, a gap 10 filled with air or another gas may be formed in the upper part of the space between the receiving portion 2 and the turbid medium 1, as indicated in the left part of Fig. 3. As another example, gas bubbles 11 may be present in the space, as schematically indicated in the right part of Fig. 3. These gas bubbles are prone to accumulate on the inner surface of the receiving portion 2. These undesired inhomogeneities 10, 11 in the space between the receiving portion 2 and the turbid medium 1 can lead to artifacts in the reconstructed image or to serious measurement errors if the actual measurement generating the measurement set of data is performed with the inhomogeneities present. In case of the undesired inhomogeneities 10, 11 described above, light will be attenuated less than expected in the region of the inhomogeneities, since, without the inhomogeneities 10, 11 present, the light would pass through the optically matching medium 4. As a result, such inhomogeneities 10, 11 can lead to intensities detected by the respective detectors 7 which are between one and two orders of magnitude higher than the intensities which would be detected without such inhomogeneities present.

In the conventional method, it is particularly disadvantageous that the presence of undesired inhomogeneities will only become clear after image reconstruction. Similar disadvantages arise in case of other technical problems.

It has been described above that the images of the interior of the turbid medium are usually analyzed long after data acquisition. In this case, reconstruction errors caused by e.g. air bubbles, other inhomogeneities or technical problems become apparent after a patient has left e.g. a hospital where the measurement is performed. The embodiment provides a method suited to provide information whether inhomogeneities were present or other technical problems have occurred immediately after the measurement step. According to the embodiment, a fast method is provided which allows detecting inhomogeneities near the cup wall (in particular air where it normally appears) based on the raw data of the measurement set of data acquired in the measurement step. The processing step of the proposed method produces an image which can be analyzed by an operator to decide if e.g. air has been present in the measurement volume during the measurement step. Alternatively, the decision can be automated using an appropriate image processing algorithm. The main advantage of the method that will be described in the following is that it can be applied immediately after the measurement step.

The method of the embodiment is based on the property that measurement data values can be compared on a point-by-point basis to reference data values acquired in a reference measurement in order to achieve information about the occurrence of technical problems. Such a point-by-point comparison can be performed very efficiently in a short time immediately after the measurement. A further aspect of the embodiment is based on the property that measurement values acquired for directly neighboring light guides connecting to the source and the respective detector are almost only influenced by the optical properties of the medium directly surrounding the associated light guides. Thus, if the measurement values for pairs of source position and detection position in which the source position and the detection position (i.e. the ends of the light guides at the side of the measurement volume) are directly adjacent are processed in a processing step, occurrence of voids in the measurement volume or other technical problems can be identified immediately after the measurement step. In particular with respect to air voids in the measurement volume, based on such processing, analysis of the optical properties near the boundary of the measurement volume, i.e. the inner wall surface of the receiving portion 2 where air accumulation is likely to occur, is possible.

According to the present embodiment, a processing and visualization method is provided which makes the presence of such inhomogeneities clearly visible and allows locating e.g. an air bubble in the measurement volume, as will be described below.

In the processing step according to the embodiment, the analysis is performed based on a comparison of reference data values from the reference measurement and measurement data values from the actual measurement step. This comparison can be performed without a need for complicated and time-consuming algorithms. If only measured values corresponding to neighboring source-detector arrangement are taken into account, the set of data is further reduced and reliable information about those locations where technical problems will most likely occur is provided.

In the processing step according to an example, a pair of corresponding measurements of the reference measurement and the actual measurement with directly neighboring source and detection positions is processed by dividing the values (measured intensities) from the actual measurement by the values (measured intensities) from the reference measurement (it should be noted that the inverse ratio could be taken as well). If the attenuation of the light used for irradiating near the source and detection positions was higher during the actual measurement step than during the reference scan, the described ratio is lower than one. If the attenuation was lower, the ratio is larger than one. In the case that the attenuation did not change, as in case of optical matching medium 4 near the associated source and detection positions, the ratio is roughly one. As a consequence, based on the ratios for a plurality of source-detection position pairs with directly neighboring source position and detection position it is possible to decide whether the attenuation coefficient near the source and detection positions (i.e. the ends of the respective light guides 5 on the side of the measurement volume) in the actual measurement step was higher or lower than the attenuation coefficient of the optically matching medium 4 in the reference measurement step. Since the optically matching medium 4 is designed to have optical properties similar to those of a typical turbid medium 1, the ratios will not significantly deviate from one in those regions in which the turbid medium 1 touches the wall of receiving portion 2 or the space between the turbid medium 1 and the wall of the receiving portion 2 is completely filled by the optically matching medium 4. However, this is not the case if inhomogeneities such as air bubbles are present. Since air has a significantly lower attenuation coefficient than the optically matching medium 4 and the turbid medium 1, in this case the ratio will be significantly larger than one. Thus, based on the ratio it is possible to decide whether air is present at the location of the respective neighboring source and detection positions.

It should be noted that, in a case in which measurements are performed for different wavelengths of the light used for irradiating the turbid medium 1, the processing step described above can be performed after each acquisition of a set of measurement data for one wavelength and before the measurement sets of data for the next wavelength is acquired. In this case, the occurrence of a technical problem can be detected at an early stage and the problem can be dealt with before the measurements for the other wavelengths are performed. Thus, further time can be saved.

In order to allow localizing the inhomogeneities in the receiving portion 2, the ratios are preferably visualized in so-called "contact maps". The name "contact maps" results from the feature that it is possible to see from the visualization whether the turbid medium 1 touches the wall of the receiving portion 2 or not. The contact map is essentially a projection of the inner surface of the receiving portion 2 on a 2-dimensional plane. For example, in the contact maps ratios for pairs of neighboring source and detection positions are projected in that location in the 2-D plane which corresponds to the center between the projected source and detection positions. For visualization of the contact map, the projected ratios can e.g. be interpolated to a Cartesian grid and the value of the ratios can be visualized by using a color scale or a grey scale.

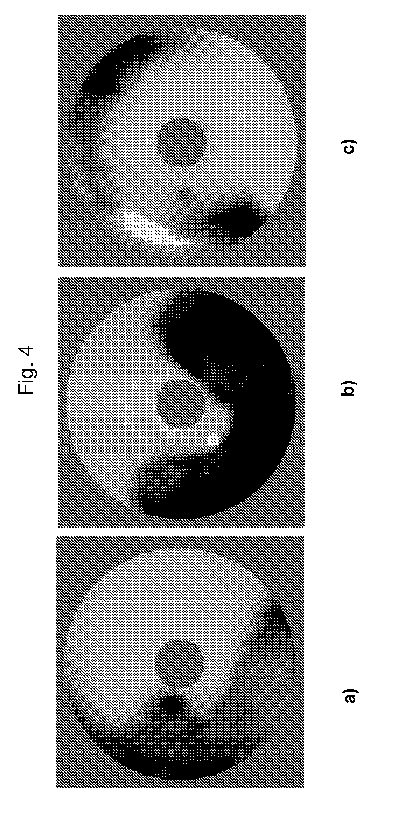

Fig. 4a to 4d show typical contact maps obtained in optical mammography for different patients. In the example, the projection is chosen such that the impression of a look into the receiving portion 2 from above arises. Fig. 4a shows an example in which the turbid medium 1 (in this case a breast) partially touches the wall of the receiving portion 2. The nearly homogeneous light-grey area is a non-contact region while the rather more inhomogeneous darker area is a contact region in which the turbid medium 1 touches the wall of the receiving portion 2. In Fig. 4b a contact map comprising an air bubble is shown. Since in this case the turbid medium has comparably high attenuation, the contact region is nearly black in this contact map. The air bubble shows as a white spot between the contact region and a non-contact region. Fig. 4c shows another example in which a larger air void is present between the receiving portion 2 and the turbid medium 1.

Further, air voids are not the only undesired artifacts that can be detected using contact maps. Technical problems can also be visualized. For, example if the optically matching medium 4 leaks between the inner wall of the receiving portion 2 and a surrounding support structure and has a negative influence on some source positions and/or detection positions, this can be clearly identified in such contact maps.

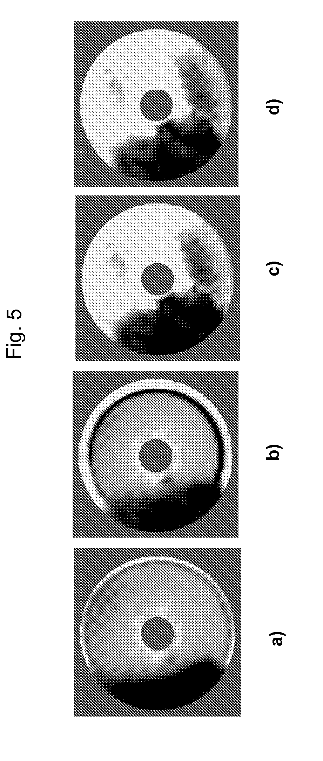

Figs. 5a to 5d show contact maps taken at different times with the time increasing from the left to the right. In this case, the level of optically matching medium 4 in the receiving portion 2 dropped with time due to a defective valve. In Fig. 5a, a thin bright ring appears at the outer rim of the contact map in the non-contact region. This ring is already thicker in Fig. 5b taken at a later time. In Figs. 5c and 5d taken at an even later time, the complete contact map is affected by the bright white regions. White regions are usually due to air at the wall of the receiving portion 2. Thus, in Fig. 5a an upper ring of source and detection positions is affected by air voids suggesting that not enough optically matching medium 4 is present in the receiving portion 2. Since more and more rings of source and detection positions (deeper in the receiving portion 2) are affected for later time points, it becomes clear that the optically matching medium leaks out of the receiving portion 2 during the measurements.

### MODIFICATIONS

Although it has been described that a plurality of light guides 5 is provided in the receiving portion for connecting to the detectors 7, the detectors 7 may also be directly arranged in the receiving portion 2 without light guides 5 interposed. Although a plurality of detectors 7 is described throughout the specification, only one detector 7 may be provided which is connected to a plurality of detection positions in the receiving portion 2, i.e. ends of light guides 5, by means of a switch. However, use of a plurality of detectors 7 is preferred.

The receiving portion 2 does not necessarily have to be formed by a cup-like receptacle as described above. It is also possible to realize the receiving portion 2 by compression surfaces between which the turbid medium is compressed. In this case, the device may comprise an optically transparent, flexible bag for accommodating the turbid medium and the optically matching medium, wherein the flexible bag and its contents, i.e. the turbid medium and the optically matching medium, are compressed between the compression surfaces. In such embodiment the surfaces are arranged mostly in parallel having a plurality of rows of light guides 5. Thus, the construction of the receiving portion could be simplified. Moreover, the position of the surfaces to each other could be made flexible. Thus, by moving the surfaces the receiving portion could be adapted to the size of turbid media.

## Claims

1. Method of detecting technical problems in a device for imaging the interior of turbid media; which device comprises:
a receiving portion (2) for receiving a turbid medium (1) to be examined;
at least one light source (6) optically connected to the receiving portion (2) for irradiating the interior of the receiving portion (2); and
at least one detector (7) optically connected to the receiving portion (2) for detecting light emanating from the interior of the receiving portion (2);
the at least one light source (6) and the at least one detector (7) being optically connected to the receiving portion (2) such that a plurality of different combinations of source position and detector position defining different light paths through the receiving portion (2) is provided;
wherein the method comprises:
a measurement step with a turbid medium (1) accommodated in the receiving portion (2); the measurement step generating measurement data consisting of a plurality of measurement data values corresponding to a plurality of different light paths through the receiving portion; and
a processing step for analyzing if technical problems have occurred during the measurement step; the processing step comprising:
comparing measurement data values to corresponding reference data values from a reference measurement with the receiving portion (2) filled with an optically matching medium.

2. The method according to claim 1, wherein comparing the measurement data values to the corresponding reference data values is performed by calculating the ratios.

3. The method according to claim 1 or 2, wherein the processing step is performed based on the measurement data before reconstruction of an image of the turbid medium (1).

4. The method according to any one of claims 1 to 3 wherein, in the processing step, only the measurement data values corresponding to directly neighboring source and detector positions are compared to corresponding reference data values.

5. The method according to any one of claims 1 to 4, wherein a graphical representation of the comparison between the measurement data values and the reference data values is provided.

6. The method according to any one of claims 1 to 5, wherein the processing step provides a result whether technical problems have occurred or not

7. The method according to any one of claims 1 to 5, wherein the processing step provides a graphical representation allowing an operator to decide if technical problems have occurred.

8. The method according to claim 5 or 7, wherein the graphical representation is provided such that it indicates at which position in the receiving portion (2) a technical problem has occurred.

9. Device for imaging the interior of turbid media, the device comprising:
a receiving portion (2) for receiving a turbid medium (1) to be examined;
at least one light source (6) optically connected to the receiving portion (2) for irradiating the interior of the receiving portion (2); and
at least one detector (7) optically connected to the receiving portion (2) for detecting light emanating from the interior of the receiving portion (2);
the at least one light source (6) and the at least one detector (7) being optically connected to the receiving portion (2) such that a plurality of different combinations of source position and detector position defining different light paths through the receiving portion (2) is provided;
wherein the device further comprises a control unit (8) which is adapted to control the device such that:
a measurement with a turbid medium (1) accommodated in the receiving portion (2) is performed; the measurement generating measurement data consisting of a plurality of measurement data values corresponding to a plurality of different light paths through the receiving portion; and
a processing operation for analyzing if technical problems have occurred during the measurement step is performed; the processing operation comprising:
comparing the measurement data values to corresponding reference data values from a reference measurement with the receiving portion (2) filled with an optically matching medium.

10. The device according to claim 9, wherein the control unit (8) is adapted such that, in the processing operation, only measurement data values corresponding to directly neighboring source and detector positions are compared.

11. Device according to claim 9 or 10, wherein the at least one light source (6) is optically connected to the receiving portion (2) such that a plurality of different source positions is distributed around the receiving portion (2).

12. Device according to any one of claims 9 to 11, wherein the at least one detector (7) is optically connected to the receiving portion (2) such that a plurality of different detection positions is distributed around the receiving portion (2).

13. Device according to any one of claims 9 to 12, wherein source positions and detection positions are alternately distributed around the receiving portion (2).

14. Device according to any one of claims 9 to 13, wherein the device is a medical image acquisition device.

15. Computer program product for a device for imaging the interior of turbid media; which device comprises:
a receiving portion (2) for receiving a turbid medium (1) to be examined;
at least one light source (6) optically connected to the receiving portion (2) for irradiating the interior of the receiving portion (2);
at least one detector (7) optically connected to the receiving portion (2) for detecting light emanating from the interior of the receiving portion (2); and
a control unit (8) controlling the operation of the device;
wherein the computer program product is adapted such that, when loaded in the control unit (8), the device is controlled such that:
a measurement step with a turbid medium (1) accommodated in the receiving portion (2) is performed; the measurement step generating measurement data consisting of a plurality of measurement data values corresponding to a plurality of different light paths through the receiving portion; and
a processing step for analyzing if technical problems have occurred during the measurement step is performed; the processing step comprising:
comparing the measurement data values to corresponding reference data values from a reference measurement with the receiving portion (2) filled with an optically matching medium.
